Europäisches Patentamt

European Patent Office

Office européen des brevets

(19)

(11) Veröffentlichungsnummer: **0 008 411**
**B1**

(12) **EUROPÄISCHE PATENTSCHRIFT**

(45) Veröffentlichungstag der Patentschrift:
27.01.82

(51) Int. Cl.³: **C 07 C 89/00, C 07 C 91/06**

(21) Anmeldenummer: 79102874.9

(22) Anmeldetag: **09.08.79**

(54) **Verfahren zur Herstellung von 5-Diäthylaminopentanol-2.**

(30) Priorität: 23.08.78 DE 2836831

(43) Veröffentlichungstag der Anmeldung:
05.03.80 Patentblatt 80/5

(45) Bekanntmachung des Hinweises auf die Patenterteilung:
27.01.82 Patentblatt 82/4

(84) Benannte Vertragsstaaten:
**BE DE FR GB NL**

(56) Entgegenhaltungen:
**FR-A-2 320 776**
**CHEMICAL ABSTRACTS, Vol. 69, 15. Juli 1968,**
**Nr, 3, Seite 948, Zusammenfassung 10063 m.**
**Columbus, Ohio, USA**
**R. S. ESKAZINA et al., »Catalytic hydrogenation**
**of acetylenic amino alcohols«**
**CHEMICAL ABSTRACTS, Vol. 71, 18. August**
**1969, Nr. 7, Seite 241, Zusammenfassung 29986 c.**
**Columbus, Ohio, USA**
**ESKAZINA, R. S. et al., »Hydrogenation of acety-**
**lenic amino alcohols«**

(73) Patentinhaber: **BASF Aktiengesellschaft,**
**Carl-Bosch-Strasse 38, D-6700 Ludwigshafen (DE)**

(72) Erfinder: **Mueller, Herbert, Dr., Carostrasse 53,**
**D-6710 Frankenthal (DE)**
Erfinder: **Toussaint, Herbert, Dr., Knietschstrasse 11,**
**D-6710 Frankenthal (DE)**
Erfinder: **Wittwer, Arnold, Dr., Londoner Ring 61,**
**D-6700 Ludwigshafen (DE)**

## Verfahren zur Herstellung von 5-Diäthylaminopentanol-2

5-Diäthylaminopentanol-2, ein wertvolles Zwischenprodukt für organische Synthesen, insbesondere auf dem pharmazeutischen Sektor, wird technisch durch Hydrierung von 1-Diäthylaminopentin-2-ol-4 hergestellt. Es ist dabei wichtig, daß eine vollständige Hydrierung der Dreifachbindung stattfindet. Acetylenische oder olefinische Verunreinigungen, die bei Verwendung von nicht sehr aktiven Katalysatoren übrig bleiben können, führen z. B. zu nicht zulässigen Verunreinigungen in den Endprodukten, aus denen sie nur schwierig entfernt werden können.

Die üblichen starken Hydrierkatalysatoren wie Platin, Palladium, Raney-Nickel usw., die für die Hydrierung von Kohlenstoff-Kohlenstoff-Doppelbindungen eingesetzt werden, werden an sich auch für die Hydrierung der Dreifachbindung zur paraffinischen Bindung verwendet. Bei der Hydrierung des Diäthylaminopentinols stellte man jedoch fest, daß gerade die sehr aktiven Platin-, Palladium-, Nickel- und Kupfer-Katalysatoren in einer Nebenreaktion Diäthylamin oder Wasser aus dem Ausgangsstoff abspalten, so daß Aminopentanol-2 bzw. Diäthylaminopentan entstehen. Da die acetylenisch ungesättigte Ausgangsverbindung verhältnismäßig wertvoll ist, sind diese Nebenreaktionen vor allem aus wirtschaftlichen Gründen nicht erwünscht und verursachen auch technische Probleme, weil deren Reaktionsprodukte abgetrennt werden müssen.

Der Erfindung liegt deshalb die Aufgabe zugrunde, ein Verfahren zur Herstellung von 5-Diäthylaminopentanol-2 durch katalytische Hydrierung von 1-Diäthylaminopentin-2-ol-4 zur Verfügung stellen, bei dem es gelingt, die Bildung von Pentanol-2 und Diäthylaminopentan zu unterdrücken und eine vollständige Reduktion zu erreichen. Die Lösung der Aufgabe gelingt dadurch, daß als Hydrier-Katalysator Nickel zusammen mit einer alklisch reagierenden Alkali- oder Erdalkali-Verbindung verwendet wird und man außerdem Wasser nach Möglichkeit ausschließt.

Aus den in C. A. 69 (1968), 948, 10063m und C. A. 71 (1969), 241, 29986c referierten Arbeiten von R. S. Eskazina et al. war es bekannt, acetylenisch ungesättigte Aminoalkohole in Gegenwart von Raney-Nickel in natronalkalischem (0.1n) Methanol und höheren Alkoholen zu hydrieren, wobei jedoch praktisch nur die olefinische Verbindung erreicht und mit steigendem Molgewicht des als Lösungsmittel dienenden Alkohols eine Abnahme der Geschwindigkeit beobachtet wurde.

Hieraus konnte nicht geschlossen werden, daß in Abwesenheit eines Lösungsmittels und möglichst wasserfreier Umsetzung ein anderer Reaktionsverlauf sich einstellen würde und statt der olefinisch ungesättigten die gesättigte Verbindung mit hoher Ausbeute erzielbar sei.

Praktisch bedeutet Wasserausschluß hier, daß der Wassergehalt im Reaktionsgemisch gering, vorzugsweise geringer als 2 Gewichtsprozent gehalten wird. Prinzipiell gilt, daß die Reaktion um so selektiver abläuft, je geringer der Wassergehalt im Reaktionsgemisch ist.

Als Lösungsmittel dient im allgemeinen das jeweilige Gemisch aus Ausgangs- und Endprodukt, d. h. fremde Lösungsmittel sind nicht erforderlich.

Besonders geeignete basische Verbindungen sind die Oxide und Hydroxide der Alkali- und Erdalkali-Metalle sowie deren Carbonate und carbonsauren Salze. Im einzelnen seien genannt Natriumhydroxid, Kaliumoxid, Calciumhydroxid, Lithiumhydroxid, Bariumhydroxid, Strontiomhydroxid, Natriumcarbonat, Natriumbicarbonat sowie Natriumacetat. In der Regel sind diese Verbindungen zwar wasserlöslich, jedoch im Reaktionsgemisch nicht oder nur wenig löslich. Galciumcarbonat ist weniger wirksam.

Die wirksame Menge an diesen Verbindungen beträgt im allgemeinen 0,02 bis 2 Gew.-%, bezogen auf die vorhandene Menge an Reaktionsgemisch, kann aber ohne Schaden auch höher liegen.

Ein besonders geeigneter Katalysator ist Raney-Nickel. Bei der Hydrierung kommt man mit katalytischen, d. h. sehr geringen Mengen Raney-Nickel aus: Im allgemeinen wird man Katalysatorkonzentrationen von 0,5 bis 5% verwenden. Eine ausreichende Reaktionsgeschwindigkeit wird bereits mit 1% Raney-Nickel erreicht. Andere geeignete Nickel-Katalysatoren sind meist handelsübliche Katalysatoren, die Nickel auf Trägern wie Kieselgel. Aluminiumoxid oder Bimsstein enthalten. Die Reaktion kann je nach der Form des Katalysators im Suspensions- oder Festbettbetrieb durchgeführt werden. Natürlich sind die vorstehenden Angaben über die Katalysatormengen nur für den Fall suspendierter Katalysatoren gedacht.

Die Reaktion verläuft bereits bei Raumtemperatur und bei gewöhnlichem Druck ausreichend schnell. Da es sich aber um eine stark exotherme Reaktion handelt, wird man im allgemeinen bei Temperaturen zwischen 50 und 100°C arbeiten, um eine ausreichende Temperaturdifferenz für eine wirksame Wärmeabfuhr zur Verfügung zu haben. In diesem Bereich ist die Reaktionstemperatur ohne Einfluß auf die Selektivität der Umsetzung, Temperaturen über 150°C sollten vermieden werden. Der angewendete Wasserstoffdruck ist nicht kritisch. Bereits bei Wasserstoffpartialdrücken unter 1 bar verläuft die Hydrierung mit hoher Geschwindigkeit. Deshalb wird man im allgemeinen schon aus Kostengründen die Anwendung von Drucken über 10 bar meiden.

## Beispiel

Ein mit einem schnellaufenden Rührwerk ausgestatteter Druckkessel von 2000 ml Nutzinhalt wird nacheinander mit 1000 g Diäthylaminopentinol (Wassergehalt ca. 0,3%), 20 g Raney-Nickel (Wassergehalt 35%) und 1,2 g Natriumhydroxid-Pulver (ca. 98%ig) beschickt. Bei einem Wasserstoffdruck von 5 bar und 19° C Ausgangstemperatur setzt sofort eine starke Reaktion ein, so daß nach 10 Minuten 50° C erreicht sind. Durch Kühlen wird die Umsetzung bei dieser Temperatur weitergeführt. Nach etwa 70 Minuten klingt die Wärmetönung ab; die Reaktion wird nun mittels Heizung bei 50° C gehalten, bis die berechnete Menge Wasserstoff aufgenommen ist (5 Stunden). Man läßt absitzen und abkühlen. Die überstehende klare Lösung wird durch ein Steigrohr abgezogen. Eine Probedestillation bei 1 mbar und 190° C zeigt, daß das Reaktionsprodukt weniger als 0,2% nicht flüchtige Bestandteile enthält. Seine Zusammensetzung in Gewichtsprozent nach gaschromatographischer Analyse gibt die folgende Tabelle an. Mit dem im Kessel verbliebenen Katalysator werden noch 4 weitere Versuche durchgeführt, deren Ergebnisse ebenfalls in der Tabelle aufgeführt sind.

Tabelle

| | Versuch 1 | 2 | 3 | 4 | 5 |
|---|---|---|---|---|---|
| Diäthylamin | 0,5 | 0,2 | 0,1 | 0,1 | 0,4 |
| Pentanol-2 | 0,3 | 0,2 | 0,3 | 0,5 | 0,3 |
| Diäthylaminopentan | 1,0 | 0,1 | 0,2 | 0,3 | 0,2 |
| Diäthylaminopentanol | 94 | 98 | 98 | 98 | 98 |
| Diäthylaminopentenol | 0,1 | – | – | – | – |
| Diäthylaminopentinol | 0,1 | – | – | – | – |
| Unbekannte Verbindungen | 3 | 1 | 1 | 0,9 | 0,9 |
| Wasser | 1 | 0,5 | 0,4 | 0,2 | 0,2 |

## Vergleichsversuch

Arbeitet man wie vorstehend jedoch ohne den Zusatz von Natriumhydroxyd-Pulver, so erhält man ein Reaktionsgemisch aus 70% Diäthylaminopentanol, ca. 10% Diäthylamin, 10% Pentanol-2 und 10% Diäthylaminopentan.

## Abwandlung des Beispiels

Die Ausbeute an Diäthylaminopentanol liegt in der gleichen Größenordnung wie im Beispiel beschrieben, also zwischen 97 und 98%, wenn man anstelle von Natriumhydroxid z. B. 1,5 g 50%ige Kalilauge verwendet. Eine Ausbeute von etwa 93% erhält man mit Lithiumhydroxid oder Calciumhydroxid als basischem Zusatz. Eine Ausbeute von 88 bis 89% beobachtet man, wenn Natriumhydroxid durch Natriumcarbonat oder Natriumbicarbonat ersetzt wird.

Mit gleichgutem Erfolg wie Raney-Nickel und Natriumhydroxid-Pulver konnte ein Katalysator verwendet werden, der nach Analyse aus 58% Nickeloxid, 15% Magnesiumoxid, 24% $SiO_2$, 1% $Cr_2O_3$ und 2% $Na_2CO_3$ besteht und vor der Benutzung bei 350—400° C reduziert wurde. Dieser Katalysator war durch Tränken eines handelsüblichen Katalysators, der Nickel auf Magnesiumsilikat als Träger enthielt mit konzentrierter Sodalösung bis zur Aufnahme von 2 Gew.-% $Na_2CO_3$ und Trocknen unter vermindertem Druck erhalten worden. Verwendet man diesen Katalysator, ohne ihn zuvor der Sodabehandlung zu unterwerfen, so erhält man das Zielprodukt mit ca. 15% geringerer Ausbeute.

Die Verwendung von Alkali- oder Erdalkaliverbindungen, die als unlöslich in Wasser gelten, wie Calcium- oder Bariumcarbonat, bietet praktisch keinen Vorteil.

## 0 008 411

### Patentansprüche

1. Verfahren zur Herstellung von 5-Diäthylaminopentanol-2 durch katalytische Hydrierung von 1-Diäthylaminopentin-2-ol-4, dadurch gekennzeichnet, daß man als Katalysator Nickel in Gegenwart einer wirksamen Menge einer basischen Alkali- oder Erdalkali-Verbindung verwendet und Wasser nach Möglichkeit ausschließt.

2. Verfahren nach Anspruch 1, dadurch gekennzeichnet, daß man die Reaktion bei Raumtemperatur beginnen läßt und eine Temperatur unter 100° C einhält.

3. Verfahren nach Anspruch 1, dadurch gekennzeichnet, daß man bei einem Wasserstoffdruck von unter 10 bar hydriert.

4. Verfahren nach Anspruch 1, dadruch gekennzeichnet, daß ein Wassergehalt von unter 2% im Reaktionsgemisch eingehalten wird.

### Claims

1. A process for the preparation of 5-diethylaminopentan-2-ol by catalytically hydrogenating 1-diethylaminopent-2-yn-4-ol, characterized in that nickel in the presence of an effictive amount of a basic alkali metal compound or alkaline earth metal compound is used as catalyst, and water is as far as possible excluded.

2. A process as claimed in claim 1, characterized in that the reaction is started at room temperature and the reaction temperature is kept below 100° C.

3. A process as claimed in claim 1, characterized in that hydrogenation is carried out under a hydrogen pressure of less than 10 bar.

4. A process as claimed in claim 1, characterized in that the water content of the reaction mixture is kept at below 2%.

### Revendications

1. Procédé pour la préparation de 5-diéthylaminopentanol-2 par hydrogénation catalytique de 1-diéthylaminopentine-2-ol-4, caractérisé en ce qu'on utilise, en tant que catalyseur, du nickel en présence d'une quantité active d'un composé alcalin ou alcalinoterreux basique et en ce qu'on exclut l'eau autant que possible.

2. Procédé selon la revendication 1, caractérisé en ce qu'on fait débuter la réaction à la température ambiante et on maintient une température inférieure à 100° C.

3. Procédé selon la revendication 1, caractérisé en ce qu'on procède à l'hydrogénation avec une pression d'hydrogène de moins de 10 bars.

4. Procédé selon la revendication 1, caractérisé en ce qu'on maintient une teneur en eau de moins de 2% dans le mélange réactionnel.

3